# EUROPÄISCHE PATENTSCHRIFT

(11) **EP 0 741 294 B1**
(45) Veröffentlichungstag und Bekanntmachung des Hinweises auf die Patenterteilung: **05.11.2003**
(21) Anmeldenummer: 96106443.3
(22) Anmeldetag: 24.04.1996
(51) Int. Cl.: G01N 33/15, G01N 33/50, G01N 33/68, G01N 33/569

(54) **Pyrogen-Untersuchungsverfahren**
Pyrogene-test-method
Procédé d'essai de pyrogénés

(30) Priorität: 03.05.1995 DE 19516247
(43) Veröffentlichungstag der Anmeldung: 06.11.1996
(73) Patentinhaber: Wendel, Albrecht, Prof. Dr., 72070 Tübingen (DE); Hartung, Thomas, Dr.rer.nat. Dr.med., 78462 Konstanz (DE)
(72) Erfinder: Wendel, Albrecht, Prof. Dr., 72070 Tübingen (DE); Hartung, Thomas, Dr.rer.nat. Dr.med., 78462 Konstanz (DE)
(74) Vertreter: TER MEER STEINMEISTER & PARTNER GbR

(56) Entgegenhaltungen:
- EP-A- 0 603 061

## Beschreibung

Die vorliegende Erfindung betrifft ein Verfahren zur Untersuchung von Stoffen auf pyrogene Wirkungen.

Es ist bekannt, daß bei Stoffen, die mit menschlichem Gewebe, Körperflüssigkeiten und Zellen in Berührung kommen, die Gefahr pyrogener Wirkungen besteht, teils weil sie als solche pyrogen sind, teils weil sie pyrogene Anteile enthalten. Zu Stoffen mit einem solchen Gefahrenspotential gehören insbesondere Medikamente, vor allem inhalierbare, injizierbare und infundierbare Produkte, einschließlich Blutersatz- und Blutaustausch-Materialien, Kunststoffe unterschiedlichster Aggregatzustände, Formen und Bestimmungen, einschließlich Dispersionen und Werkstoffen wie Membranen und Prothesen bis hin zu kosmetischen Produkten. Selbst Nahrungsmittel können eine Gefährdung durch Pyrogene darstellen Stoffe, die im Kontakt mit menschlichem Gewebe, Zellen oder Körperflüssigkeiten pyrogen wirken, werden üblicherweise als Stoffe mit exogenen pyrogenen Wirkungen bezeichnet, unabhängig von der möglicherweise selbst endogenen Herkunft des pyrogenen Wirkanteils des jeweiligen Stoffs. So ist auch nachfolgend der Begriff der exogenen Pyrogene bzw. der Stoffe mit exogenen pyrogenen Wirkungen, mit deren Untersuchung sich die vorliegende Erfindung befaßt, zu verstehen.

Die Problematik der Pyrogentestung ist bekannt. Die Produktsicherheit ist ein dringendes Bedürfnis und macht daher, um auch ausnahmsweise kontaminierte Chargen zu erkennen, ständige Einzelprüfungen an Versuchstieren erforderlich.

Als wichtigste fiebererzeugende Komponente gilt Endotoxin (Lipopolysaccharid, LPS) aus der Bakterienwand Gram-negativer Keime (Moltz, H. , Neurosci. Biobehav. Rev. (1993), 17, 237-269; Tilders et al., Psychoneuroendocrinology, 1994, 19, 209-232; Rothwell, Crit.Rev.Neurobiol., 1994, 8, 1 - 10; Zeisberger und Roth, Neuropsychobiology, 1993, 28, 106-109). Es lag deshalb nahe, den Tierversuch, der im allgemeinen am Kaninchen durchgeführt wird, durch eine direkte Endotoxinbestimmung (Limulus-Test, LAL) zu ersetzen. Dieser Ansatz ist naturgemäß beschränkt: Es werden nur Endotoxine als potentielle Fieberauslöser erfaßt; andere Stimulatoren der Leukozyten, die ebenfalls zu Fieber führen, entziehen sich der Erfassung. Der Test ist sehr störanfällig gegenüber Endotoxin-bindenden Komponenten wie sie z.B. im Blut und Blutkomponenten reichlich vorkommen (Harris et al., J.Lab.Clin.Med. 1991, 118, 186-193; Emancipator et al., Infect. Immun. 60, 1992, 596-601. Read et al., Eur.Heart J., 1993, 14, 125-129); einige dieser Endotoxin-bindenden Komponenten entziehen das LPS der Messung im Limulus-Test während sie im Gegenteil die Reaktion mit Leukozyten, also die eigentliche pyrogene Primärreaktion verstärken. Gerade die Pyrogenbestimmung in Blutprodukten ist jedoch ein häufiger Testfall. Der Limulus-Test ist so empfindlich, daß es sehr leicht durch Verunreinigungen, die nicht für die Produktqualität relevant sind, zu Fehlbestimmungen kommen kann (Fujiwara et al., Yakugaku Zasshi, 1990, 110, 332-340).

Andererseits ist auch der Kaninchenversuch nicht unproblematisch. Die Immunantwort (Fieber) auf einen gegebenen Reiz unterscheidet sich von Spezies zu Spezies erheblich. Inwieweit das Kaninchen für den Menschen, selbst auch für Tiere, repräsentativ ist, ist durchaus fraglich, da z.B. die Endotoxin-Empfindlichkeit verschiedener Spezies um den Faktor 10.000 variieren kann. Für andere pyrogene Komponenten gibt es keine vergleichbaren Untersuchungen. EP 0 603 061 offenbart ein Verfahren zur Untersuchung eines Stoffes auf pyrogene Wirkung in einer medizinischen und biologischen lösung.

Aufgabe der vorliegenden Erfindung ist es, ein Verfahren zur Verfügung zu stellen, welches die Erkennung pyrogener Wirkstoffe auf großer Bandbreite ermöglicht. Das Verfahren sollte einfach und ohne großen Kostenaufwand durchführbar sein. Weitere Aufgaben ergeben sich nachfolgend, insbesondere aus den geltendgemachten Vorteilen.

Die Lösung der gestellten Aufgabe gelingt auf dem aus den Patentansprüchen ersichtlichem Wege.

Der besondere Vorteil dieses Verfahrens besteht in der Verwendung eines biologischen Systems, das relevante Aussagen für die Exposition des Menschen liefert. Als Meßparameter dienen die endogenen Pyrogene. Diese sind Botenstoffe des Immunsystems, die die Fieberreaktion vermitteln und sozusagen die Mitteilung des Organismus, daß ein Pyrogen erkannt wurde, darstellen. Insbesondere handelt es sich dabei z.B. um Zytokine bzw. Wachstumsfaktoren. Die wichtigsten bekannten endogenen Pyrogene sind die Proteine Interleukin-1 (IL-1), Interleukin-6 (IL-6) und der Tumor-Nekrose-Faktor (TNF) sowie niedermolekulare Lipidmediatoren wie Prostaglandin E₂ (PGE₂). Deren Bestimmung kann in üblicher Weise z.B. für IL-1, IL-6 oder TNF im ELISA (Enzyme-Linked Immunosorbent Assay) oder für PGE₂ z.B. mit EIA (Enzyme Immuno Assay) erfolgen.

Als biologisches System wäre auch die Verwendung von aus Blut isolierten Leukozyten möglich. Die meisten Isolierungs-Prozeduren sind jedoch für eine Routinetestung von Substanzen sehr aufwendig. Einfacher ist es deshalb, tierisches oder menschliches Vollblut ohne weitere Trennung von Einzelkomponenten wie frisch gewonnenes Blut gesunder menschlicher Spender für diesen Zweck zu verwenden. Die Leukozyten liegen so in ihrer natürlichen Zusammensetzung und Umgebung vor. Gleichzeitig sind alle Serumkomponenten anwesend, die auf die Wirkung eines Pyrogens Einfluß haben könnten. Dabei ist es von besonderem Vorteil, daß dem Vollblut gerinnungsverzögernde oder gerinnungshindernde Bestandteile wie Citrat, so z.B. in einer Endkonzentration von 0,38 % oder Heparin wie Na-Heparin z.B. zur Gerinnungshemmung auch während der Inkubation zugesetzt werden können, ohne daß solche Bestandteile die erfindungsgemäße Reaktion beeinträchtigen oder gar verfälschen.

Sehr bewährt hat sich auch eine Verdünnung der Vollblutzubereitung, vor allem mit Zellkulturmedium, beispielsweise eine 20%ige Verdünnung mit physiologischer Kochsalzlösung. Dabei versteht es sich von selbst, daß alle Gerätschaften, wie Laborgeräte und Zusatzstoffe, wie Gerinnungshemmer oder Verdünnungsmittel möglichst pyrogenfrei sind und auch während der Inkubation, wo vorsorglich auch Antibiotika, wie Penicillin oder Streptomycin zugegen sein können, unter Bedingungen gearbeitet wird, die eine relevante Kontamination ausschließen.

Als Beispiele für die Freisetzung endogener Pyrogene können die vier vorgenannten endogenen Pyrogene, die durch verschiedene Bakterienbestandteile von Gram-negativen und Gram-positiven Bakterien freigesetzt werden, dienen. Das Verfahren ist gegenüber Endotoxinen hoch empfindlich (wenige pg/ml führten zur Freisetzung von endogenen Pyrogenen) und weitgehend unabhängig vom Blutspender. Zur Positivkontrolle können Präparationen von Gram-negativen und Gram-positiven Bakterienwänden wie Endotoxin und Lipoteichonsäure, zur Negativkontrolle z.B. pyrogenfreie physiologische Kochsalzlösung verwendet werden.

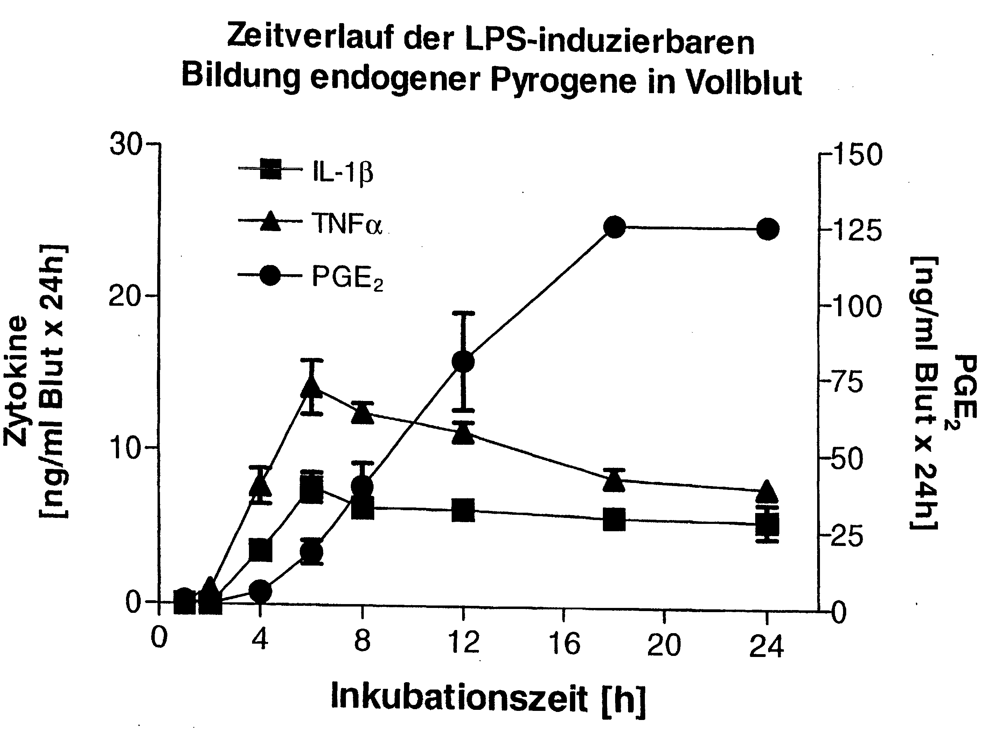

Zur Bestimmung des Zeitverlaufs der Endotoxin-induzierten Bildung endogener Pyrogene im Vollblut wurde Blut von 7 gesunden Spendern mit Zellkulturmedium 1:5 verdünnt und mit 10 µg/ml LPS stimuliert (Abb.1). Alle Faktoren waren mit kommerziellen ELISAs (Mittelwerte ± SEM) nach Inkubation (37°C) nachweisbar, während unstimuliertes Blut diese Faktoren nicht freisetzte. Die gefundene IL-1β-Menge erreichte nach ca. 6h ein Plateau. PGE₂ nahm bis 24h Inkubationszeit kontinuierlich zu. Ebenso stieg die induzierbare IL-6-Menge (gemessen nach 4, 10 und 24h stets an - Daten nicht gezeigt). Hingegen zeigt die TNF-Freisetzung nach LPS-Stimulation ein Maximum bei ca. 12h. Da jedoch auch zum Zeitpunkt 24h noch der größte Teil der TNF-Menge vorhanden war, wurde dies als Standardinkubationszeit gewählt.

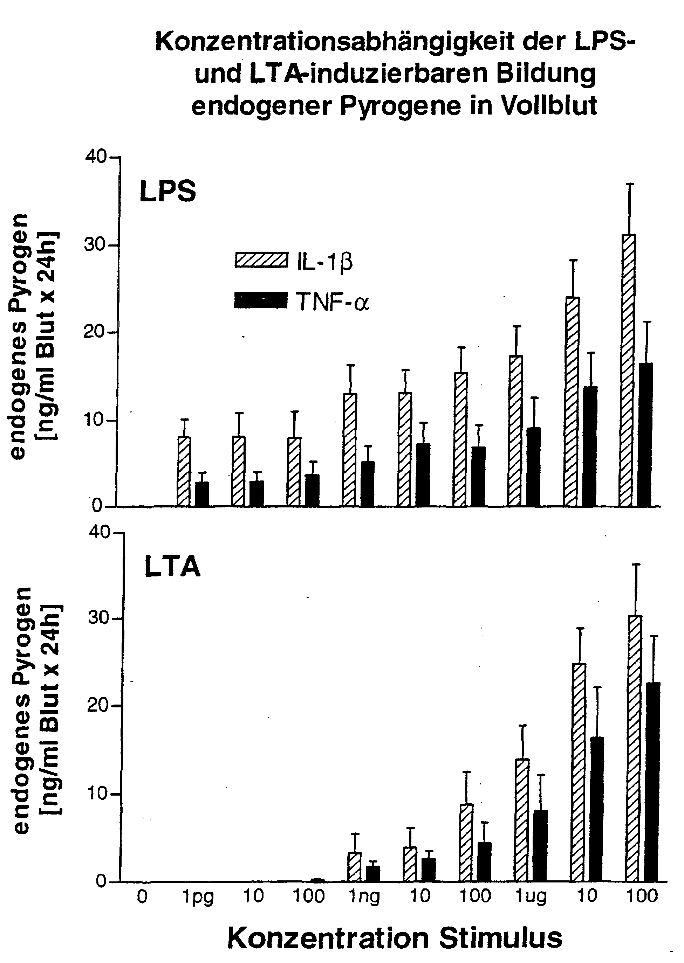

Zur Bestimmung der Konzentrationsabhängigkeit der Gram-negativen Endotoxin- und Gram-positiven Lipoteichonsäure-induzierten Bildung endogener Pyrogene in Vollblut wurde Blut von 5 gesunden Spendern 1:5 verdünnt und für 24h in Gegenwart der angegebenen Endotoxin- bzw. Lipoteichonsäure-Konzentration inkubiert. Die Cytokin-Bestimmung erfolgte im ELISA. Die Daten sind Mittelwerte ± SEM (Abb.2). Tatsächlich führte bereits die kleinste eingesetzte LPS-Konzentration von 1 pg/ml zu einer signifikanten Bildung dieser endogenen Pyrogene, während der Zusatz des LPS- bzw. LTA-Lösungsmittels ohne Effekt war. Es zeigt sich, daß menschliches Vollblut sehr empfindlich und konzentrationsabhängig auf LPS und LTA reagiert.

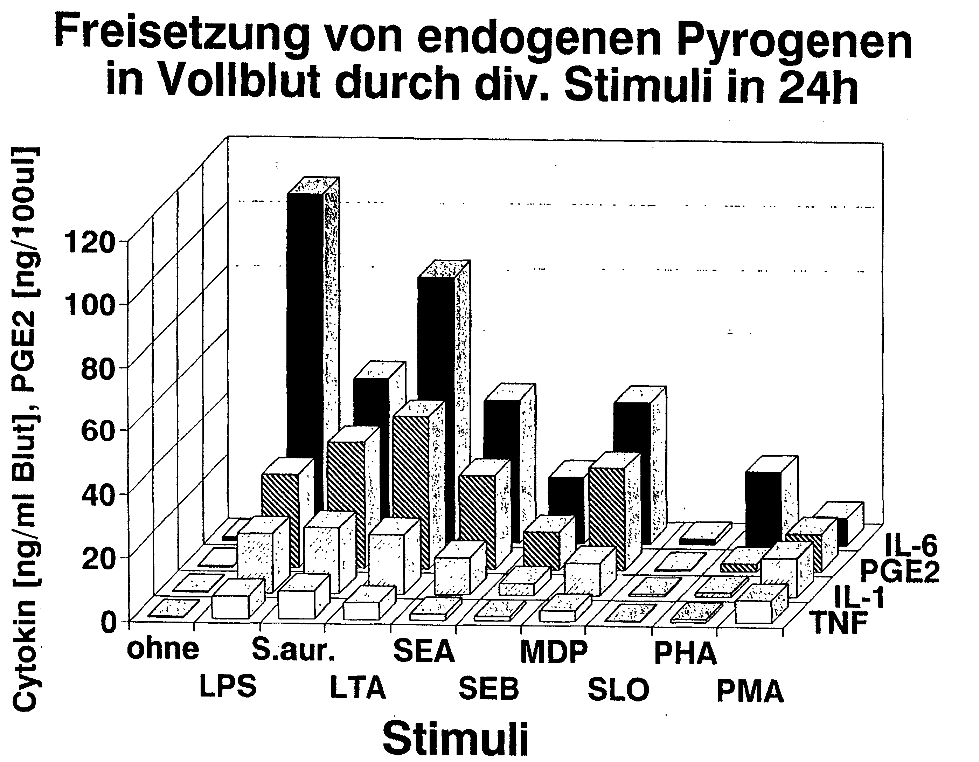

Auch andere Immunstimulatoren als die vorgenannten werden als Freisetzer von endogenen Pyrogenen bei diesem Verfahren erfaßt. So führten auch hitzegetötete Gram-positive Bakterien (Staphylococcus aureus) oder deren Bestandteile (Muropeptid, Lipoteichonsäure, Enterotoxine, Streptolysin 0) zu einer ähnlichen Reaktion (Abb.3). Auch andere Immunstimulatoren wie der Pflanzeninhaltsstoff Phytohemagglutinin oder Phorbolester lösten eine entsprechende Mediatorfreisetzung aus.

Das Verfahren weist eine ganze Reihe von Vorteilen auf. Es wird die körpereigene Primärreaktion der Bildung von endogenen Pyrogenen auf die Gabe von exogenen Pyrogenen zur Untersuchung herangezogen. Es sind alle Blutkomponenten vorhanden, die ggf. für eine Interaktion des exogenen Pyrogens mit den Leukozyten notwendig sind (z.B. LPS-bindendes Protein LBP, bakterizides Permeabilitäts-erhöhendes Protein BPI, lösliches CD14, Defensine etc.). Die tierischen oder menschlichen Blutzellen liegen in ihrer natürlichen Mischung und Umgebung vor. Es entstehen kaum Präparationsartefakte wie bei isolierten Leukozyten und Dedifferenzierungen wie bei Zellinien. Das.Verfahren ist sehr sensitiv (z.B. pg-Mengen von LPS). Es werden eine Vielzahl von potentiellen Pyrogenen neben Endotoxinen erfaßt. Es können auch pharmakologische fiebersenkende Wirkungen in-vitro oder ex-vivo geprüft werden.

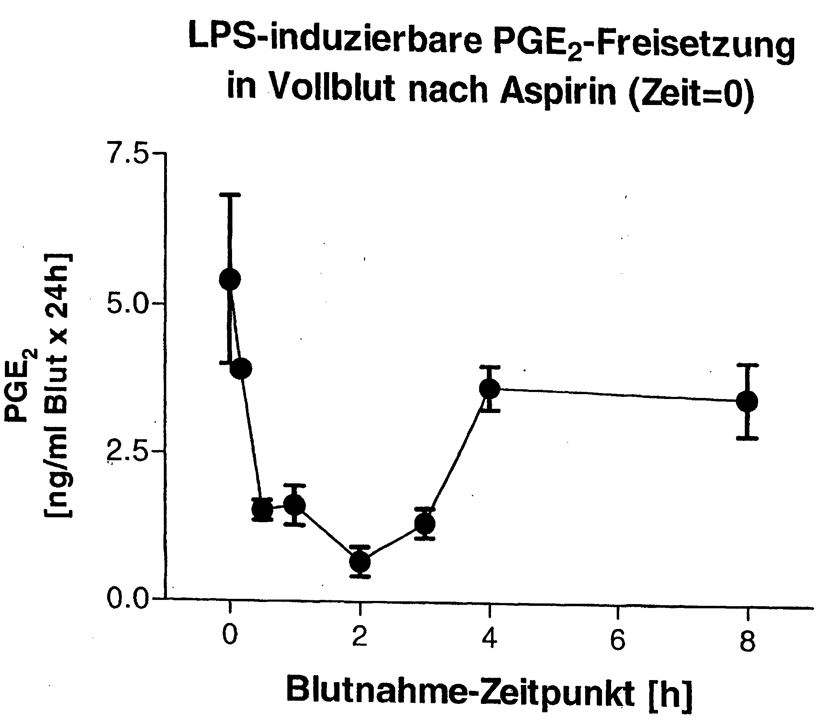

So eignet sich das Verfahren auch dazu, daß als Prüfung die Auswirkung einer fiebersenkenden Therapie bei Menschen, aber auch eine fiebersenkende Wirkung bei Tieren, nachvollzogen werden kann. Drei gesunde Probanden erhielten hierzu zum Zeitpunkt O je ein fiebersenkendes Mittel, im Beispiel je eine Tablette Aspirin (500 mg) und es wurde Blut unmittelbar vorher sowie zu verschiedenen Zeiten danach abgenommen. Tatsächlich nahm die mit einem exogenen Pyrogen, im Beispiel mit 10 µg/ml LPS-induzierbare PGE₂-Menge in Folge der Einnahme des fiebersenkenden Medikamentes rasch ab (Abb.4) und erholte sich erst nach einigen Stunden. Die PGE₂-Menge wurde durch EIA bestimmt (Mittelwerte ± SEM).

### BEISPIEL

Zitratblut gesunder Spender wurde unmittelbar nach Abnahme mit Zellkulturmedium RPMI 1640 (Biochrom, Berlin) 1:5 verdünnt. Heparin [2 IE/ml als Endkonzentration] (Liquemin^{R}, Hoffmann LaRoche, Grenzach-Whylen) wurde dem System zugesetzt, um eine Gerinnung durch Recalciflzierung auszuschließen. Stimuli wurden zeitgleich zugesetzt bzw. bereits mit dem Zellkulturmedium vorgelegt. Im einzelnen wurden folgende Substanzen in den angegebenen maximalen Konzentrationen verwendet: LPS von Salmonella abortus equi [10 µg/ml],Enterotoxin A (SEA) und B (SEB) von Staphylococcus aureus [1 µg/ml], Lipoteichonsäure (LTA) von S. aureus [10 µg/ml], hitze-getötete S. aureus [0.001 % Zellen v/v], Streptolysin 0 (SLO) von Streptococcus pyogenes [2.5 Einheiten/ml], Muramyldipeptid (MDP) [10 µg/ml], Phytohemagglutinin M (PHA) [15 µg/ml] und Phorbolester (PMA) [100nM]. Alle Stimuli außer MDP (Bachem, Heidelberg) wurden von Sigma (Deisenhofen) bezogen. Die Inkubationen wurden in offenen Polypropylen-Reaktionsgefäßen (Eppendorf, Hamburg) bei 37°C und 5 % C02 für standardmäßig 24h durchgeführt (Wilson, et al., J.Immunol. Meth., 1991, 139, 233-240). Die zellfreien Überstände wurden nach Zentrifugation (3000g, 1 min) abgenommen und bei -80°C bis zur Cytokin- bzw. Prostaglandinmessung aufbewahrt.

Die freigesetzten Mediatoren wurden mit kommerziellen ELISAs gemessen: IL-1, IL-6 und TNF wurden mit Assays von R & D Systems (H. Biermann, Bad Nauheim) bestimmt, während für PGE₂ ein Assay von Cayman (SPI-Bio Europe, Frankreich) verwendet wurde.

## Patentansprüche

1. Verfahren zur Untersuchung von Stoffen und Werkstoffen, die mit menschlichem Gewebe, Körperflüssigkeiten und Zellen in Berührung kommen, auf pyrogene Wirkungen unterschiedlicher Ursachen, mit folgenden Schritten
- die zu untersuchenden Stoffe und Werkstoffe werden mit menschlicher oder tierischer Vollblut-Zubereitung in Kontakt gebracht;
- danach erfolgt eine Inkubation der Vollblut-Zubereitung unter die Bildung endogener Pyrogene ermöglichenden Bedingungen, **dadurch gekennzeichnet, daß** die Vollblut-Zubereitung gerinnungsverzögernde oder -verhindernde Anteile enthält und
- die so inkubierte Vollblut-Zubereitung auf die Bildung von Zytokinen untersucht wird.

2. Verfahren gemäß Patentanspruch 1, **dadurch gekennzeichnet, daß** die Vollblut-Zubereitung Verdünnungsmittel enthält.

3. Verfahren gemäß Patentanspruch 2, **dadurch gekennzeichnet, daß** Zellkulturmedium oder physiologische Kochsalzlösung als Verdünnungsmittel enthalten sind.

4. Verfahren gemäß einem der vorhergehenden Patentansprüche, **dadurch gekennzeichnet, daß** auf die Bildung von Interleukin-1, -6, TNF und/oder Prostaglandin E2 geprüft wird.

## Claims

1. Method for investigating substances and materials which come into contact with human tissue, body fluids and cells for pyrogenic effects of various causes, with the following steps:
- the substances and materials to be investigated are brought into contact with human or animal whole blood preparation;
- after which the whole blood preparation is incubated under conditions making the production of endogenous pyrogens possible, **characterized in that** the whole blood preparation contains coagulation-delaying or -preventing portions, and
- the whole blood preparation incubated in this way is investigated for the production of cytokines.

2. Method according to Claim 1, **characterized in that** the whole blood preparation contains diluent.

3. Method according to Claim 2, **characterized in that** cell culture medium or physiological saline are present as diluent.

4. Method according to any of the preceding claims, **characterized in that** the production of interleukin-1, -6, TNF and/or prostaglandin E2 is tested.

## Revendications

1. Procédé pour tester des corps et matières qui viennent en contact avec des cellules, des liquides corporels et des tissus humains, en ce qui concerne les effets pyrogènes de diverses origines, comprenant les étapes suivantes :
- on met en contact les corps et substances à tester avec une préparation de sang complet humain ou animal ;
- après cela se produit une incubation de la préparation de sang complet, dans des conditions permettant la formation de pyrogènes endogènes, **caractérisé en ce que** :
- la préparation de sang complet contient des ingrédients inhibiteurs ou retardateurs de la coagulation ; et
- on teste la préparation de sang complet ainsi incubée en ce qui concerne la formation de cytokines.

2. Procédé selon la revendication 1, **caractérisé en ce que** la préparation de sang complet contient du solvant.

3. Procédé selon la revendication 2, **caractérisé en ce qu'**en tant qu'un milieu de culture cellulaire ou une solution de sel de cuisine physiologique sont contenus en tant que solvant.

4. Procédé selon l'une des revendications précédentes, **caractérisé en ce qu'**on fait des essais en ce qui concerne interleukine-1, -6, TNF et/ou prostaglandine E2.
